(19) Europäisches Patentamt
European Patent Office
Office européen des brevets

(11) Numéro de publication: 0 071 520
B1

# (12) FASCICULE DE BREVET EUROPEEN

(45) Date de publication du fascicule du brevet: **04.09.85**

(51) Int. Cl.⁴: **C 07 D 401/04**, A 61 K 31/00

(21) Numéro de dépôt: **82401357.7**

(22) Date de dépôt: **21.07.82**

(54) **Nouveaux dérivés du pipéridin-3-yl indole, leurs sels, leur procédé de préparation, leur application à titre de médicaments et les compositions pharmaceutiques les renfermant.**

(30) Priorité: **24.07.81 FR 8114428**

(43) Date de publication de la demande: **09.02.83 Bulletin 83/6**

(45) Mention de la délivrance du brevet: **04.09.85 Bulletin 85/36**

(84) Etats contractants désignés: **AT BE CH DE GB IT LI LU NL SE**

(56) Documents cités:
**EP-A-0 021 924**
**FR-A-2 391 211**
**GB-A-1 030 623**
**GB-A-1 224 530**
**GB-A-2 055 815**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Nedelec, Lucien, 45, boulevard de L'Ouest, F-93340-Le Raincy (FR)**
Inventeur: **Guillaume, Jacques, 15, avenue du Belvédère App. 1904, F-93310-Le Pré Saint Gervais (FR)**
Inventeur: **Dumont, Claude, 33, rue du Maréchal Vaillant, F-94130 Nogent Sur Marne (FR)**

(74) Mandataire: **Petranker, Léon et al, boîte postale no 9 102, route de Noisy, F-93230 Romainville (FR)**

EP 0 071 520 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés du pipéridin-3-yl indole ainsi que leurs sels, leur procédé de préparation, leur application à titre de médicaments et les compositions pharmaceutiques les renfermant.

Des dérivés du pipéridin- et du tétrahydropyridin--3-yl indole doués notamment de propriétés dopaminergiques centrales ont déjà été décrits dans la demande de brevet européen n° 0.021.924. La présente demande concerne des dérivés du 2-oxo indole doués de propriétés dopaminergiques périphériques.

L'invention a pour objet de nouveaux dérivés du pipéridin-3-yl indole, ainsi que leurs sels d'addition avec les acides minéraux ou organiques, caractérisés en ce qu'ils répondent à la formule générale I:

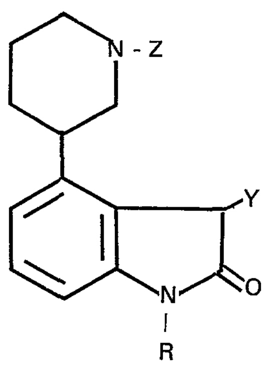

(I)

dans laquelle:

— R représente un atome d'hydrogène, un radical alkyle renfermant de 1 à 8 atomes de carbone ou un radical aralkyle renfermant de 7 à 12 atomes de carbone,

— Y représente un radical alkyle renfermant de 1 à 8 atomes de carbone,

— Z représente un atome d'hydrogène, un radical méthyle, un radical alkyle ou hydroxyalkyle renfermant de 2 à 8 atomes de carbone, un radical aryloxyalkyle renfermant de 7 à 12 atomes de carbone, un radical aralkyle renfermant de 7 à 12 atomes de carbone, éventuellement substitué par un ou plusieurs atomes d'halogène, par un ou plusieurs radicaux alkoxy renfermant de 1 à 4 atomes de carbone, par un ou plusieurs radicaux alkyles renfermant de 1 à 4 atomes de carbone ou par un ou plusieurs radicaux OH, $CF_3$, $OCF_3$, $NO_2$ ou $NH_2$ ou Z représente un radical cycloalkylalkyle renfermant de 4 à 12 atomes de carbone, un radical alkényle renfermant de 3 à 8 atomes de carbone ou un radical alkynyle renfermant de 3 à 8 atomes de carbone.

Les sels d'addition avec les acides minéraux ou organiques peuvent être, par exemple, les sels formés avec les acides chlorhydrique, bromhydrique, nitrique, sulfurique, phosphorique, acétique, propionique, formique, benzoïque, maléïque, fumarique, succinique, tartrique, citrique, oxalique, glyoxylique, aspartique, alcanesulfoniques tels que l'acide méthane sulfonique et aryl sulfoniques, tels que l'acide benzène sulfonique.

Lorsque R représente un radical alkyle, il s'agit de préférence du radical méthyle, éthyle, propyle, isopropyle, butyle, isobutyle ou n-pentyle.

Lorsque R représente un radical aralkyle, il s'agit de préférence du radical benzyle.

Lorsque Y représente un radical alkyle, il s'agit de préférence d'un radical alkyle renfermant de 1 à 4 atomes de carbone et notamment du radical méthyle, éthyle, n-propyle ou n-butyle.

Lorsque Z représente un radical alkyle ou hydroxy alkyle, «alkyle» représente de préférence un radical éthyle, n-propyle ou isopropyle, n-butyle ou isobutyle, n-pentyle ou n-hexyle.

Lorsque Z représente un radical aryloxyalkyle, il s'agit de préférence du radical phényloxyéthyle ou phényloxypropyle.

Lorsque Z représente un radical aralkyle, il s'agit de préférence d'un radical benzyle ou phénéthyle.

Lorsque Z représente un radical aralkyle substitué par un ou plusieurs atomes d'halogène, on entend de préférence par halogène un atome de chlore ou de brome.

Lorsque Z représente un radical aralkyle substitué par un ou plusieurs radicaux alkyle, on entend de préférence par alkyle, un radical méthyle ou éthyle.

Lorsque Z représente un radical aralkyle substitué par un ou plusieurs radicaux alkoxy, on entend de préférence par alcoxy un radical méthoxy ou éthoxy.

Lorsque Z représente un radical cycloalkylalkyle, il s'agit de préférence d'un radical cyclopropylalkyle, par exemple, du radical cyclopropylméthyle, cyclopropyléthyle, ou cyclopropyle n-propyle.

Lorsque Z représente un radical alkényle, il s'agit de préférence du radical allyle.

Lorsque Z représente un radical alkynyle, il s'agit de préférence du radical propargyle.

Parmi les produits, objet de l'invention, on peut citer notamment, les dérivés répondant à la formule I ci-dessus ainsi que leurs sels d'addition avec les acides minéraux ou organiques caractérisés en ce que, dans ladite formule I, Y représente un radical méthyle.

Parmi ceux-ci, on peut citer plus particulièrement les dérivés caractérisés en ce que, dans ladite formule (I) Z représente un atome d'hydrogène ou un radical alkyle renfermant de 1 à 8 atomes de carbone, et tout particulièrement:

— la 1,3-dihydro-3-méthyl-4-(pipéridin-3-yl)2H indole-2-one, ainsi que ses sels d'addition avec les acides minéraux ou organiques.

L'invention a également pour objet un procédé de préparation des dérivés, tels que définis par la formule I ci-dessus, ainsi que de leurs sels, caractérisé en ce que l'on soumet un produit de formule II

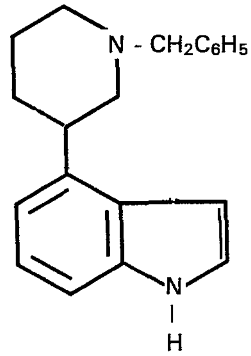

(II)

a une alkylation, pour obtenir un produit de formule $I_A$:

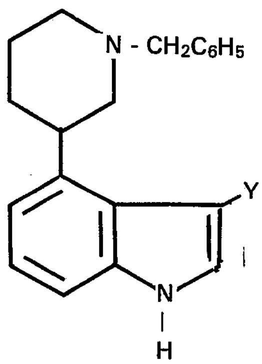

($I_A$)

dans laquelle Y a la signification déjà indiquée, que si désiré l'on fait réagir avec un halogénure de formule III:

$$Hal_1 - R' \qquad (III)$$

dans laquelle $Hal_1$ représente un atome de chlore, de brome ou d'iode, et R' a la signification de R à l'exception d'hydrogène, pour obtenir un produit de formule $I_C$:

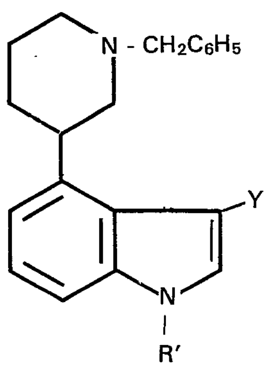

($I_C$)

dans laquelle R' et Y ont la signification déjà indiquée, puis soumet lesdits produits de formules $I_A$ et $I_C$ à l'action d'un agent de clivage du groupement benzyle porté par l'azote du cycle pipéridinyle, pour obtenir un produit de formule $I_E$:

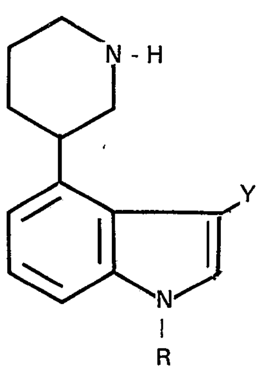

($I_E$)

dans laquelle R et Y ont la signification déjà indiquée, produit de formule $I_E$ que l'on soumet à l'action d'un agent oxydant, pour obtenir un produit de formule $I_I$:

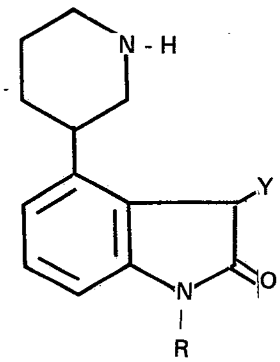

($I_I$)

dans laquelle R et Y ont la signification déjà indiquée, produit de formule $I_I$ que, ou bien l'on isole et salifie si désiré ou bien l'on soumet à l'action d'un agent capable d'introduire un radical Z', Z' ayant la signification de Z à l'exception de l'hydrogène pour obtenir un produit de formule $I_J$:

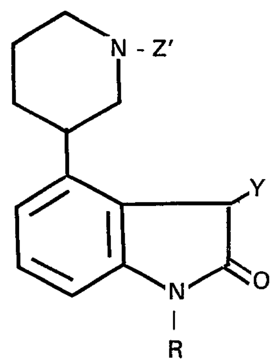

($I_J$)

dans laquelle R, Y et Z' ont la signification déjà indiquée, que l'on isole et salifie si désiré.

L'alcoylation du produit de formule II peut être réalisée, par exemple, selon la réaction de Mannich ou encore à l'aide d'une formylation de Vilsmeier-Haack, notamment à l'aide de diméthyl formamide et d'oxychlorure de phosphore, suivie de la réduction du dérivé formylé obtenu lorsque l'on veut obtenir un dérivé méthylé.

Pour l'obtention des homologues supérieurs, on peut faire réagir le dérivé formylé ci-dessus, avec un phosphorane de formule générale $(C_6H_5)_3 P = CH - Y'$, dans laquelle Y' représente un atome d'hydrogène, ou un radical alcoyle renfermant de 1 à 6 atomes de carbone puis hydrogéner le dérivé alcénylé obtenu. La réaction du produit de formule $I_A$ avec l'halogénure de formule III est avantageusement réalisée en présence d'agents fixateurs d'acides, tels que des carbonates ou hydroxydes alcalins, comme ceux de sodium ou de potassium ou des amines tertiaires.

L'halogénure de formule III est de préférence un iodure.

L'agent de clivage du groupement benzyle porté par l'azote du cycle pipéridinyle est l'hydrogène en présence d'un catalyseur, de préférence le palladium.

Lorsque l'on veut méthyler le dérivé de formule II, on peut avantageusement effectuer une réaction de Mannich; dans ce cas, le clivage du benzyle porté par

l'azote du cycle pipéridinyle se fait en même temps que celui de l'amine de l'aminométhyle, par hydrogénolyse.

L'introduction du radical Z' est réalisée au moyen d'un halogénure Z'-Hal, dans lequel Hal a la signification déjà indiquée; cet halogénure réagit avantageusement dans les conditions décrites pour l'halogénure de formule III.

L'halogénation des produits de formule $I_A$, $I_C$ et $I_E$ peut être réalisée par exemple à l'aide du complexe bromé de la pyridine de formule 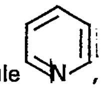, $Br_2$, HBr dans le cas de la bromation. Elle est réalisée avantageusement à l'aide d'un N-halosuccinimide, de préférence le N-bromo ou le N-chloro.

L'agent oxydant est de préférence le mélange diméthylsulfoxyde -HCl concentré.

L'invention a aussi pour objet une variante du procédé ci-dessus décrit, pour la préparation des dérivés, tels que définis par la formule I ci-dessus, ainsi que leurs sels, caractérisé en ce que l'on soumet le produit de formule $I_E$ à l'action d'un agent capable d'introduire un radical Z', Z' ayant la signification de Z à l'exception d'hydrogène, pour obtenir un produit de formule $I_G$:

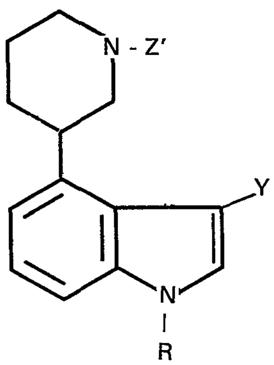

($I_G$)

dans laquelle R, Y et Z' ont la signification déjà indiquée, que l'on soumet à l'action d'un agent oxydant, pour obtenir le produit de formule $I_J$.

L'agent oxydant utilisé est identique à celui cidessus décrit.

Les dérivés de formule I présentent un caractère basique. On peut avantageusement préparer les sels d'addition des dérivés de formule I, en faisant réagir, en proportions sensiblement stoechiométriques, un acide minéral ou organique avec ledit dérivé de formule I. Les sels peuvent être préparés sans isoler les bases correspondantes.

Les dérivés objet de la présente invention, possèdent de très intéressantes propriétés pharmacologiques; ils sont doués notamment de remarquables propriétés stimulantes dopaminergiques, illustrées plus loin dans la partie expérimentale. Ils possèdent en outre des propriétés antianoxiques.

Ces propriétés justifient l'utilisation des dérivés du pipéridin-3-yl indole de formule I ainsi que de leurs sels, pharmaceutiquement acceptables, à titre de médicaments.

La présente demande a ainsi également pour objet l'application, à titre de médicaments, des dérivés du pipéridin-3-yl indole, tels que définis par la formule I, ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés du pipéridin-3-yl indole, répondant à la formule I, dans laquelle Y représente un radical méthyle ainsi que par leurs sels d'addition avec les acides pharmaceutiquement acceptables.

Parmi ceux-ci, on retient notamment ceux répondant à la formule I, dans laquelle Z représente un atome d'hydrogène ou un radical alkyle renfermant de 1 à 8 atomes de carbone, ainsi que leurs sels d'addition avec les acides pharmaceutiquement acceptables.

Parmi ces derniers, on retient tout particulièrement:

— la 1,3-dihydro-3-méthyl-4-(pipéridin-3-yl)2H-indole-2-one, ainsi que ses sels d'addition avec les acides pharmaceutiquement acceptables.

Les médicaments selon l'invention trouvent leur emploi par exemple dans le traitement de très nombreuses maladies ou désordres pathologiques divers, ils peuvent, par exemple, être utilisés dans le traitement de l'hypersecrétion de prolactine par l'antéhypophyse, et de ce fait, dans le traitement de l'hypogonadisme de la femme ou de l'homme, ils peuvent également être utilisés dans le traitement des syndromes neurologiques d'origine extrapyramidale, par exemple dans le traitement de la maladie de Parkinson et dans le traitement des syndromes parkinsoniens post-encéphalitiques.

Ils peuvent être également utilisés dans le traitement de la sénescence cérébrale et en gériatrie.

La dose usuelle, variable selon le dérivé utilisé, le sujet traité et l'affection en cause, peut être, par exemple de 5 mg à 100 mg par jour, par voie orale chez l'homme du dérivé de l'exemple 2, pour le traitement de la maladie de Parkinson.

L'invention a aussi pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme, par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de départ de formules II et III sont connus; ils peuvent être préparés comme indiqué dans le brevet français n° 2 458 549 ou la demande de brevet européen n° 0 021 924.

Les exemples qui suivent illustrent l'invention sans toutefois la limiter.

*Exemple 1: 1,3-dihydro-3-méthyl-4-(pipéridin-3-yl)2H-indol-2-one*

*Stade A: N,N-diméthyl-4-(1-phénylméthyl pipéridin-3-yl)1H-indole-3-méthanamine*

On ajoute lentement sous agitation à 0° - 5°C, 2,5 $cm^3$ d'acide acétique, puis 1,4 $cm^3$ d'une solution aqueuse d'aldéhyde formique à 40%, sous atmosphère inerte, à 2,5 $cm^3$ d'une solution aqueuse de diméthylamine à 35%, puis une solution de 5,5 g de 4-[1-(phénylméthyl) pipéridin-3-yl]1H-indole dans 25 $cm^3$ d'acide acétique, laisse pendant 1h 30 sous agitation à température ambiante, dilue à l'eau, alcalinise à l'aide de lessive de soude, filtre, lave à l'eau, sèche sous pression réduite à 60°C, et obtient après recristallisation dans le benzène, 3 g du produit attendu. F ≃ 150°C.

*Stade B: Chlorhydrate de 3-méthyle-4-(pipéridin-3-yl)1H-indole*

On hydrogène à 40°C, 4,3 g de produit tel qu'obtenu ci-dessus dans 300 $cm^3$ de méthanol, sous agitation, en présence de 1,5 g de palladium à 10% sur charbon. Après 1 heure, on ajoute 1,5 g de palladium à 10% sur charbon et poursuit l'hydrogénation. Après encore 1 heure, on filtre, chasse les solvants à 50°C sous pression réduite et obtient 2,5 g de base du produit attendu. F = 150°C.

*Formation de chlorhydrate:*

On dissout le produit ci-dessus dans 250 $cm^3$ d'acétate d'éthyle, ajoute à 0°C/5°C, une solution d'acétate d'éthyle chlorhydrique jusqu'à pH acide, filtre, lave à l'acétate d'éthyle, sèche à 50°C sous pression réduite, recristallise dans l'isopropanol et obtient 2,4 g du produit attendu. F = 280°C.

*Analyse:* $C_{14}H_{19}ClN_2$ = 250,773

| Calculé: | Trouvé: |
|---|---|
| C % = 67,05 | 66,7 |
| H % = 7,64 | 7,6 |
| N % = 11,17 | 11,1 |
| Cl % = 14,14 | 14,1. |

*Stade C: 1,3-dihydro-3-méthyl-4-(pipéridin-3-yl)2H-indol-2-one*

On agite pendant 30 minutes à température ambiante, sous atmosphère inerte, 14 $cm^3$ de diméthylsulfoxyde et 32 $cm^3$ d'acide chlorhydrique 22° Bé avec 5 g du chlorhydrate de l'exemple n° 1, dilue à l'eau, ajoute de la lessive de soude jusqu'à pH alcalin, extrait à l'acétate d'éthyle, lave à l'eau puis avec une solution aqueuse d'hydroxyde de sodium, sèche, chasse le solvant à 40°C sous pression réduite, purifie par chromatographie sur silice (éluant: chloroforme-acétone-triéthylamine 6-3-1) et isole les fractions de Rf. = 0,1. On obtient 3,1 g du produit attendu.

*Spectre U.V.:* dans EtOH
— max 250 nm $E^{1\%}_{1\,cm}$ = 336 ε = 7700
— max 280 nm $E^{1\%}_{1\,cm}$ = 58 ε = 1300

*Spectre U.V.:* dans EtOH/NaOH N/10
— max 263-264 nm $E^{1\%}_{1\,cm}$ = 352 ε = 8100
— inf 274 $E^{1\%}_{1\,cm}$ = 287
— inf 290 $E^{1\%}_{1\,cm}$ = 84

*Spectre IR* ($CHCl_3$)
— 3440 $cm^{-1}$ NH(=C - NH + associé)
— 1715 $cm^{-1}$ C = O (γ-lactame)
— 1620 - 1600 - 1490 $cm^{-1}$: aromatiques.

*Exemple 2: 1,3-dihydro-3-méthyl-4(pipéridin-3-yl)2H-indol-2-one*

*Stade A: 2-bromo-3-méthyl-4-(pipéridin-3-yl)1H-indole*

On agite pendant 20 heures à température ambiante, sous atmosphère inerte 250 mg de chlorhydrate du stade B de l'exemple n° 1 dans 10 $cm^3$ de dioxane, avec 178 mg de N-bromosuccinimide.

On reprend à l'eau, alcalinise, extrait à l'acétate d'éthyle, sèche, chasse le solvant à 40°C sous pression réduite, purifie par chromatographie sur silice (éluant: chloroforme-acétone-triéthylamine 6-3-1) et obtient 120 mg de produit attendu.

*Analyse:*

*Spectre U.V.* dans l'éthanol:
— max 224 nm $E^{1\%}_{1\,cm}$ = 1100 ε = 32250
— infl 276 nm $E^{1\%}_{1\,cm}$ = 291
— max 280 nm $E^{1\%}_{1\,cm}$ = 300 ε = 8800
— infl 289 nm $E^{1\%}_{1\,cm}$ = 244.

*Stade B: 1,3-dihydro-3-méthyl-4(pipéridin-3-yl)2H-indol-2-one*

On agite pendant une heure à température ambiante 120 mg du produit de l'exemple 2 en solution dans l'acide chlorhydrique N, isole le produit obtenu comme indiqué à l'exemple 1 et obtient 47 mg du produit attendu.

*Etude pharmacologique*

*Inhibition de la prolactine plasmatique in vivo*

On place des rats mâles de souche Sprague-Dawley par deux dans des cages et les met pendant une semaine dans une pièce insonorisée à température contrôlée à 22 ± 2°C, soumise à un nycthémère artificiel de 14 heures de jour et 10 heures de nuit. On place sous anesthésie un cathéter dans la veine cave supérieure droite des rats, puis leur administre 48 heures après, une injection intrapéritonéale de 5 mg/kg de réserpine, suivie d'une administration orale de 5 mg/kg du produit testé.

La prolactine plasmatique est mesurée par la méthode radio-immunologique décrite par Euvrard et al. [Neuropharmacology, 19, 379 (1980)], sur des prélèvements sanguins de 0,7 ml.

Les résultats sont exprimés en durée d'inhibition de la prolactine plasmatique.

Les résultats obtenus sont les suivants:

Le produit des exemples 1 et 2 inhibe la prolactine plasmatique pendant une durée d'environ 15 heures.

*Inhibition de la secrétion de prolactine effectuée sur des cellules de rat in vitro*

On prépare des cultures primaires de cellules pituitaires antérieures de rat, selon la technique décrite par Drouin et Labrie [Endocrinology 98, 1528 (1976)]. Après 4 heures d'incubation avec le produit testé ou sans produit pour les témoins, on mesure la prolactine présente dans le milieu par la méthode radio-immunologique décrite par Euvrard et al. [Neuropharmacology, 19, 379 (1980)].

Les résultats sont exprimés en DE 50: dose inhibant 50% de la secrétion de prolactine par comparaison aux témoins.

Les résultats obtenus sont les suivants:

La DE50 des produits des exemples 1 et 2 est égale à 10 nMoles.

*Etude de la toxicité aiguë*

On a évalué la dose létale $DL_O$ des composés des exemples 1 et 2 après administration par voie orale chez la souris.

On appelle $DL_O$ la dose maximale ne provoquant aucune mortalité au bout de 8 jours.

Les résultats obtenus ont été les suivants:

Produit des exemples 1 et 2: $DL_O$ = 200 mg/kg.

**Revendications pour les Etats contractants:**
BE, CH, DE, GB, IT, LI, LU, NL, SE

1. Nouveaux dérivés du pipéridin-3-yl indole ainsi que leurs sels d'addition avec les acides minéraux ou organiques, caractérisés en ce qu'ils répondent à la formule générale I:

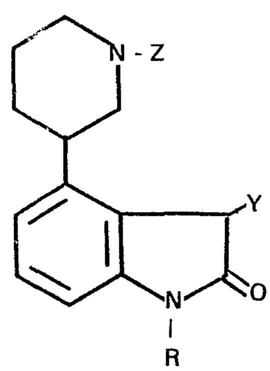

(I)

dans laquelle:

— R représente un atome d'hydrogène, un radical alkyle renfermant de 1 à 8 atomes de carbone ou un radical aralkyle renfermant de 7 à 12 atomes de carbone,

— Y représente un radical alkyle renfermant de 1 à 8 atomes de carbone,

— Z représente un atome d'hydrogène, un radical méthyle, un radical alkyle ou hydroxyalkyle renfermant de 2 à 8 atomes de carbone, un radical aryloxyalkyle renfermant de 7 à 12 atomes de carbone, un radical aralkyle renfermant de 7 à 12 atomes de carbone éventuellement substitué par un ou plusieurs atomes d'halogène, par un ou plusieurs radicaux alkoxy renfermant de 1 à 4 atomes de carbone, par un ou plusieurs radicaux alkyle renfermant de 1 à 4 atomes de carbone ou par un ou plusieurs radicaux OH, $CF_3$, $OCF_3$, $NO_2$ ou $NH_2$ ou Z représente un radical cycloalkylalkyle renfermant de 4 à 12 atomes de carbone, un radical alkényle renfermant de 3 à 8 atomes de carbone ou un radical alkynyle renfermant de 3 à 8 atomes de carbone.

2. Nouveaux dérivés du pipéridin-3-yl indole répondant à la formule I telle que définie à la revendication 1 ainsi que leurs sels d'addition avec les acides minéraux ou organiques, caractérisés en ce que Y représente un radical méthyle.

3. Nouveaux dérivés du pipéridin-3-yl indole selon la revendication 2 ainsi que leurs sels d'addition avec les acides minéraux ou organiques, caractérisés en ce que Z représente un atome d'hydrogène ou un radical alkyle renfermant de 1 à 8 atomes de carbone.

4. La 1,3-dihydro 3-méthyl 4-(pipéridin-3-yl)2H indole-2-one, ainsi que ses sels d'addition avec les acides minéraux ou organiques.

5. Procédé de préparation des dérivés du pipéridin-3-yl indole de formule I telle que définie à la revendication 1 ainsi que de leurs sels d'addition avec les acides minéraux ou organiques caractérisé en ce que l'on soumet un produit de formule II:

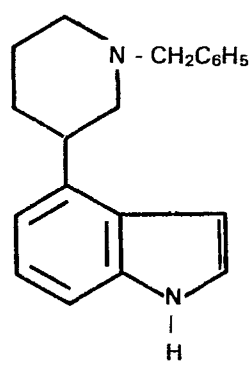

(II)

à une alkylation, pour obtenir un produit de formule $I_A$:

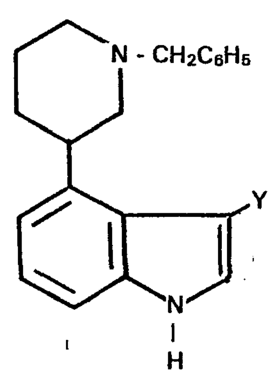

($I_A$)

dans laquelle Y a la signification déjà indiquée, que si désiré l'on fait réagir avec un halogénure de formule III:

$Hal_1$ - R'

dans laquelle $Hal_1$ représente un atome de chlore, de brome ou d'iode, et R' a la signification de R à l'exception d'hydrogène, pour obtenir un produit de formule $I_C$:

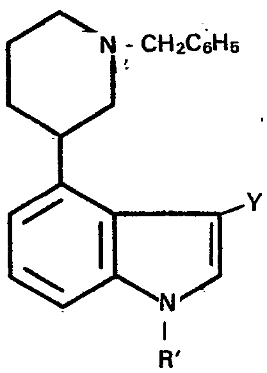

($I_C$)

dans laquelle R' et Y ont la signification déjà indiquée, puis soumet lesdits produits de formules $I_A$ et $I_C$ à l'action d'un agent de clivage du groupement benzyle porté par l'azote du cycle pipéridinyle, pour obtenir un produit de formule $I_E$:

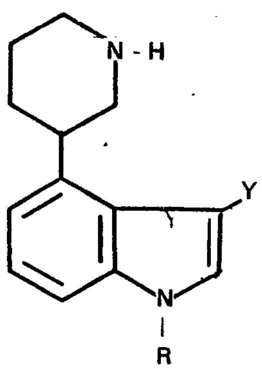

($I_E$)

dans laquelle R et Y ont la signification déjà indiquée, produit de formule $I_E$ que l'on soumet à l'action d'un agent oxydant, pour obtenir un produit de formule $I_I$:

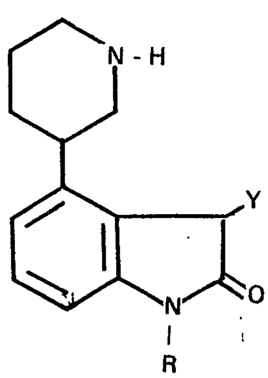

($I_I$)

dans laquelle R et Y ont la signification déjà indiquée, produit de formule $I_I$ que, ou bien l'on isole et salifie si désiré ou bien l'on soumet à l'action d'un agent capable d'introduire un radical Z', Z' ayant la signification de Z à l'exception de l'hydrogène pour obtenir un produit de formule $I_J$:

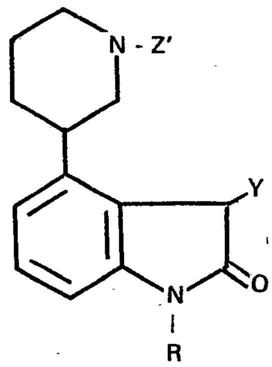

($I_J$)

dans laquelle R, Y et Z' ont la signification déjà indiquée, que l'on isole et salifie si désiré.

6. Procédé selon la revendication 5, caractérisé en ce que:
— l'agent oxydant est le mélange diméthylsulfoxyde -HCl concentré.

7. Variante du procédé de la revendication 5, caractérisé en ce que l'on soumet le produit de formule $I_E$ à l'action d'un agent capable d'introduire un radical Z', Z' ayant la signification de Z à l'exception d'hydrogène, pour obtenir un produit de formule $I_G$:

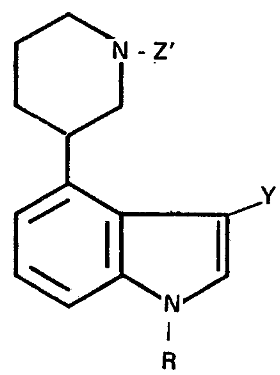

($I_G$)

dans laquelle R, Y et Z' ont la signification déjà indiquée, que l'on soumet à l'action d'un agent oxydant, pour obtenir le produit de formule $I_J$.

8. Médicaments, caractérisés en ce qu'ils sont constitués par les nouveaux dérivés du pipéridin-3-yl indole, tels que définis par la formule I de la revendication 1 ainsi que par leurs sels d'addition avec les acides pharmaceutiquement acceptables.

9. Médicaments, caractérisés en ce qu'ils sont constitués par les nouveaux dérivés du pipéridin-3-yl indole, tels que définis dans l'une des revendications 2 ou 3, ainsi que par leurs sels d'addition avec les acides pharmaceutiquement acceptables.

10. Médicaments, caractérisés en ce qu'ils sont constitués par le dérivé du pipéridin-3-yl indole, tel que défini à la revendication 4 ainsi que par ses sels d'addition avec les acides pharmaceutiquement acceptables.

11. Compositions pharmaceutiques, caractérisées en ce qu'elles renferment, à titre de principe actif, l'un au moins des médicaments, tels que définis à l'une des revendications 8, 9 ou 10.

**Revendications pour l'Etat contractant:** AT

1. Procédé pour préparer les nouveaux dérivés du pipéridin-3-yl indole ainsi que leurs sels d'addition avec les acides minéraux ou organiques, répondant à la formule générale I:

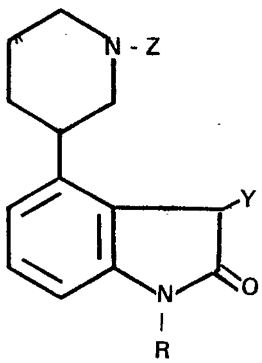

(I)

dans laquelle:
— R représente un atome d'hydrogène, un radical alkyle renfermant de 1 à 8 atomes de carbone ou un radical aralkyle renfermant de 7 à 12 atomes de carbone,
— Y représente un radical alkyle renfermant de 1 à 8 atomes de carbone,
— Z représente un atome d'hydrogène, un radical méthyle, un radical alkyle ou hydroxyalkyle renfermant de 2 à 8 atomes de carbone, un radical aryloxyalkyle renfermant de 7 à 12 atomes de carbone, un radical aralkyle renfermant de 7 à 12 atomes de carbone éventuellement substitué par un ou plusieurs atomes d'halogène, par un ou plusieurs radicaux alkoxy renfermant de 1 à 4 atomes de carbone, par un ou plusieurs radicaux alkyle renfermant de 1 à 4 atomes de carbone ou par un ou plusieurs radicaux OH, $CF_3$, $OCF_3$, $NO_2$ ou $NH_2$ ou Z représente un radical cycloalkylalkyle renfermant de 4 à 12 atomes de carbone, un radical alkényle renfermant de 3 à 8 atomes de carbone ou un radical alkynyle renfermant de 3 à 8 atomes de carbone, caractérisé en ce que l'on soumet un produit de formule II:

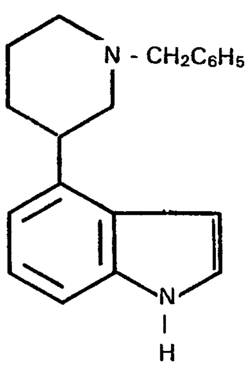

(II)

à une alkylation, pour obtenir un produit de formule $I_A$:

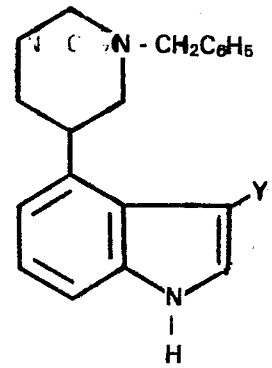

($I_A$)

dans laquelle Y a la signification déjà indiquée, que si désiré l'on fait réagir avec un halogénure de formule III:

$Hal_1$ - R'

dans laquelle $Hal_1$ représente un atome de chlore, de brome ou d'iode, et R' a la signification de R à l'exception d'hydrogène, pour obtenir un produit de formule $I_C$:

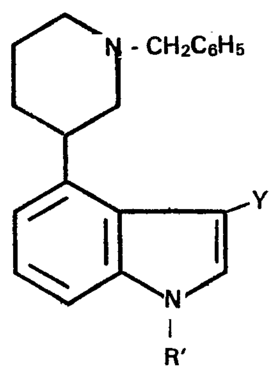

($I_C$)

dans laquelle R' et Y ont la signification déjà indiquée, puis soumet lesdits produits de formules $I_A$ et $I_C$ à l'action d'un agent de clivage du groupement benzyle porté par l'azote du cycle pipéridinyle, pour obtenir un produit de formule $I_E$:

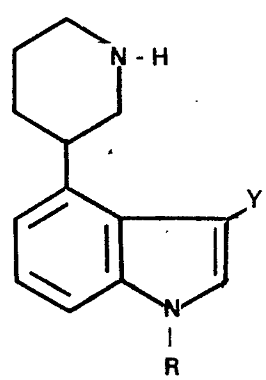

($I_E$)

dans laquelle R et Y ont la signification déjà indiquée, produit de formule $I_E$ que l'on soumet à l'action d'un agent oxydant, pour obtenir un produit de formule $I_I$:

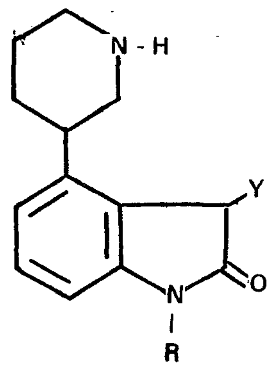

$(I_I)$

dans laquelle R et Y ont la signification déjà indiquée, produit de formule $I_I$ que, ou bien l'on isole et salifie si désiré ou bien l'on soumet à l'action d'un agent capable d'introduire un radical Z', Z' ayant la signification de Z à l'exception de l'hydrogène pour obtenir un produit de formule $I_J$:

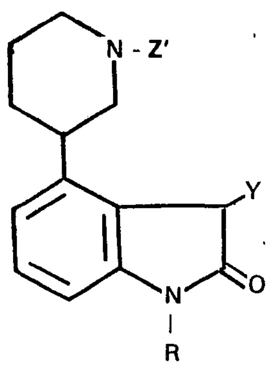

$(I_J)$

dans laquelle R, Y et Z' ont la signification déjà indiquée, que l'on isole et salifie si désiré.

2. Procédé selon la revendication 1, caractérisé en ce que:

— l'agent oxydant est le mélange diméthylsulfoxyde -HCl concentré.

3. Variante du procédé de la revendication 1, caractérisé en ce que l'on soumet le produit de formule $I_E$ à l'action d'un agent capable d'introduire un radical Z', Z' ayant la signification de Z à l'exception d'hydrogène, pour obtenir un produit de formule $I_G$:

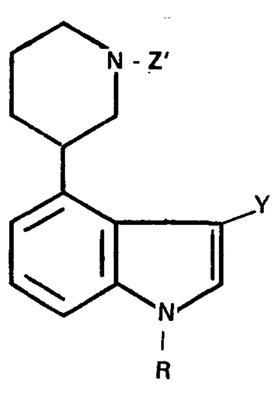

$(I_G)$

dans laquelle R, X', Y et Z' ont la signification déjà indiquée, que l'on soumet à l'action d'un agent oxydant, pour obtenir le produit de formule $I_J$.

4. Procédé selon l'une quelconque des revendications 1 à 3, caractérisé en ce que l'on prépare des dérivés de formule I, ainsi que leurs sels d'addition avec les acides minéraux ou organiques, formule I dans laquelle Y représente un radical méthyle.

5. Procédé selon l'une quelconque des revendications 1 à 4, caractérisé en ce que l'on prépare des dérivés de formule I, ainsi que leurs sels d'addition avec les acides minéraux ou organiques, formule I dans laquelle Z représente un atome d'hydrogène ou un radical alkyle renfermant de 1 à 8 atomes de carbone.

6. Procédé selon l'une quelconque des revendications 1 à 3, caractérisé en ce que l'on prépare la 1,3-dihydro 3-méthyl 4-(pipéridin-3-yl) 2H indole-2-one, ainsi que ses sels d'addition avec les acides minéraux ou organiques.

**Patentansprüche für die Vertragsstaaten:**
BE, CH, DE, GB, IT, LI, LU, NL, SE

1. Neue Piperidin-3-yl-indol-Derivate sowie deren Additionssalze mit Mineral- oder organischen Säuren, dadurch gekennzeichnet, dass sie der allgemeinen Formel I

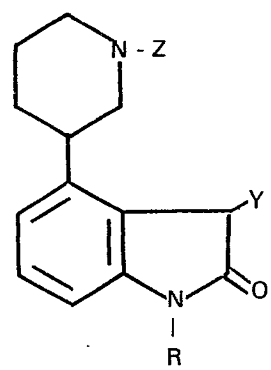

(I)

entsprechen, worin

R ein Wasserstoffatom, einen Alkylrest mit 1 bis 8 Kohlenstoffatomen oder einen Aralkylrest mit 7 bis 12 Kohlenstoffatomen bedeutet,

Y einen Alkylrest mit 1 bis 8 Kohlenstoffatomen bedeutet,

Z ein Wasserstoffatom, einen Methylrest, einen Alkyl- oder Hydroxyalkylrest mit 2 bis 8 Kohlenstoffatomen, einen Aryloxyalkylrest mit 7 bis 12 Kohlenstoffatomen, einen Aralkylrest mit 7 bis 12 Kohlenstoffatomen, der gegebenenfalls durch ein oder mehrere Halogenatome, durch einen oder mehrere Alkoxyreste mit 1 bis 4 Kohlenstoffatomen, durch einen oder mehrere Alkylreste mit 1 bis 4 Kohlenstoffatomen oder durch einen oder mehrere Reste OH, $CF_3$, $OCF_3$, $NO_2$ oder $NH_2$ substituiert ist, bedeutet oder Z einen Cycloalkylalkylrest mit 4 bis 12 Kohlenstoffatomen, einen Alkenylrest mit 3 bis 8 Kohlenstoffatomen oder einen Alkinylrest mit 3 bis 8 Kohlenstoffatomen bedeutet.

2. Neue Piperidin-3-yl-indol-Derivate der Formel I gemäss Anspruch 1 sowie deren Additionssalze mit Mineral- oder organischen Säuren, dadurch gekennzeichnet, dass Y einen Methylrest bedeutet.

3. Neue Piperidin-3-yl-indol-Derivate gemäss Anspruch 2 sowie deren Additionssalze mit Mineral- oder organischen Säuren, dadurch gekennzeichnet, dass Z ein Wasserstoffatom oder einen Alkylrest mit 1 bis 8 Kohlenstoffatomen bedeutet.

4. 1,3-Dihydro-3-methyl-4-(piperidin-3-yl)-2H--indol-2-on sowie dessen Additionssalze mit Mineral- oder organischen Säuren.

5. Verfahren zur Herstellung der Piperidin-3-yl--indol-Derivate der Formel I gemäss Anspruch 1 sowie von deren Additionssalzen mit Mineral- oder organischen Säuren, dadurch gekennzeichnet, dass man ein Produkt der Formel II

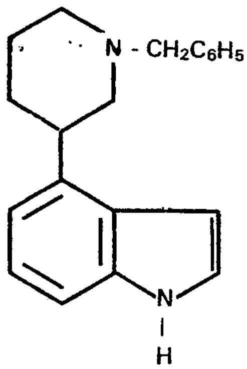

(II)

einer Alkylierung unterzieht, um ein Produkt der Formel $I_A$

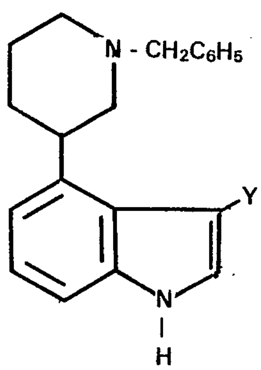

($I_A$)

zu erhalten, worin Y die angegebene Bedeutung besitzt, welches man gewünschtenfalls mit einem Halogenid der Formel III

$Hal_1$ - R'

umsetzt, worin $Hal_1$ ein Chlor-, Brom- oder Jodatom bedeutet und R' die Bedeutung von R mit Ausnahme von Wasserstoff besitzt, um ein Produkt der Formel $I_C$

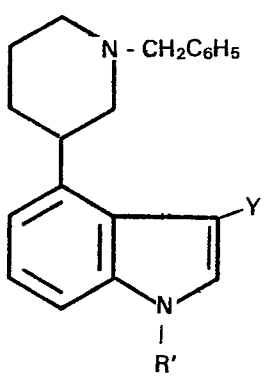

($I_C$)

zu erhalten, worin R' und Y die angegebene Bedeutung besitzen, danach die Produkte der Formeln $I_A$ und $I_C$ der Einwirkung eines Mittels zur Abspaltung der von dem Stickstoff des Piperidinylrings getragenen Benzylgruppe unterzieht, um ein Produkt der Formel $I_E$

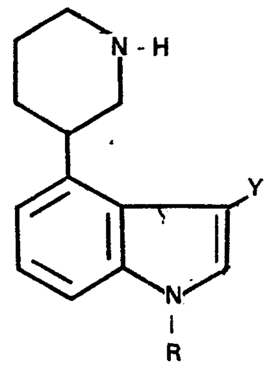

($I_E$)

zu erhalten, worin R und Y die angegebene Bedeutung besitzen, das Produkt der Formel $I_E$ der Einwirkung eines Oxidationsmittels unterzieht, um ein Produkt der Formel $I_I$

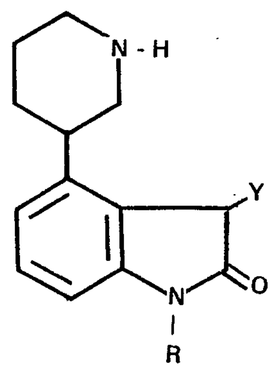

($I_I$)

zu erhalten, worin R und Y die angegebene Bedeutung besitzen, das Produkt der Formel $I_I$ entweder isoliert und gewünschtenfalls in ein Salz überführt oder der Einwirkung eines Mittels, das befähigt ist, einen Rest Z' einzuführen, unterzieht, wobei Z' die Bedeutung von Z mit Ausnahme von Wasserstoff besitzt, um ein Produkt der Formel $I_J$

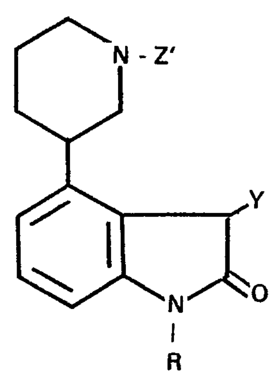

($I_J$)

zu erhalten, worin R, Y und Z' die angegebene Bedeutung besitzen, das man isoliert und gewünschtenfalls in ein Salz überführt.

6. Verfahren gemäss Anspruch 5, dadurch gekennzeichnet, dass
das Oxidationsmittel ein Dimethylsulfoxid-konzentrierte HCl-Gemisch ist.

7. Abänderung des Verfahrens nach Anspruch 5, dadurch gekennzeichnet, dass man das Produkt der Formel $I_E$ der Einwirkung eines Mittels unterzieht, das befähigt ist, einen Rest Z' einzuführen, wobei Z' die Bedeutung von Z mit Ausnahme von Wasserstoff besitzt, um ein Produkt der Formel $I_G$

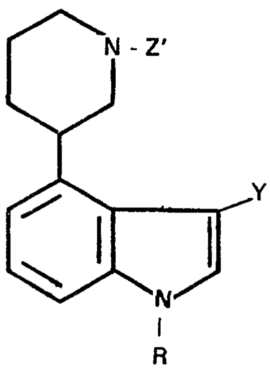

$(I_G)$

zu erhalten, worin R, Y und Z' die angegebene Bedeutung besitzen, das man der Einwirkung eines Oxidationsmittels unterzieht, um das Produkt der Formel $I_J$ zu erhalten.

8. Arzneimittel, dadurch gekennzeichnet, dass sie aus den neuen Piperidin-3-yl-indol-Derivaten der Formel I gemäss Anspruch 1 sowie aus deren Additionssalzen mit pharmazeutisch verträglichen Säuren bestehen.

9. Arzneimittel, dadurch gekennzeichnet, dass sie aus den neuen Piperidin-3-yl-indol-Derivaten gemäss einem der Ansprüche 2 oder 3 sowie aus deren Additionssalzen mit pharmazeutisch verträglichen Säuren bestehen.

10. Arzneimittel, dadurch gekennzeichnet, dass sie aus dem Piperidin-3-yl-indol-Derivat gemäss Anspruch 4 sowie aus dessen Additionssalzen mit pharmazeutisch verträglichen Säuren bestehen.

11. Pharmazeutische Zusammensetzungen, dadurch gekennzeichnet, dass sie als Wirkstoff zumindest eines der Arzneimittel gemäss einem der Ansprüche 8, 9 oder 10 enthalten.

**Patentansprüche für den Vertragsstaat:** AT

1. Verfahren zur Herstellung von neuen Piperidin-3-yl-indol-Derivaten sowie von deren Additionssalzen mit Mineral- oder organischen Säuren, der allgemeinen Formel I:

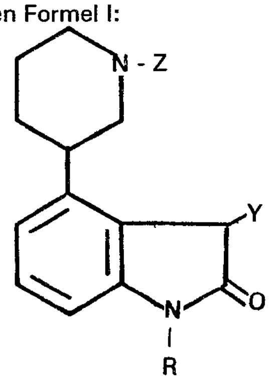

(I)

worin

R ein Wasserstoffatom, einen Alkylrest mit 1 bis 8 Kohlenstoffatomen oder einen Aralkylrest mit 7 bis 12 Kohlenstoffatomen bedeutet,
Y einen Alkylrest mit 1 bis 8 Kohlenstoffatomen bedeutet,
Z ein Wasserstoffatom, einen Methylrest, einen Alkyl- oder Hydroxyalkylrest mit 2 bis 8 Kohlenstoffatomen, einen Aryloxyalkylrest mit 7 bis 12 Kohlenstoffatomen, einen Aralkylrest mit 7 bis 12 Kohlenstoffatomen, der gegebenenfalls durch ein oder mehrere Halogenatome, durch einen oder mehrere Alkoxyreste mit 1 bis 4 Kohlenstoffatomen, durch einen oder mehrere Alkylreste mit 1 bis 4 Kohlenstoffatomen oder durch einen oder mehrere Reste OH, $CF_3$, $OCF_3$, $NO_2$ oder $NH_2$ substituiert ist, bedeutet, oder Z einen Cycloalkylalkylrest mit 4 bis 12 Kohlenstoffatomen, einen Alkenylrest mit 3 bis 8 Kohlenstoffatomen oder einen Alkinylrest mit 3 bis 8 Kohlenstoffatomen bedeutet, dadurch gekennzeichnet, dass man ein Produkt der Formel II:

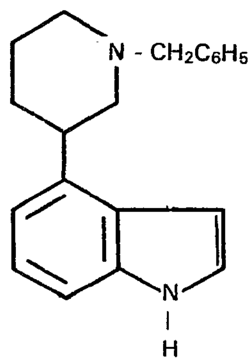

(II)

einer Alkylierung unterzieht, um ein Produkt der Formel $I_A$

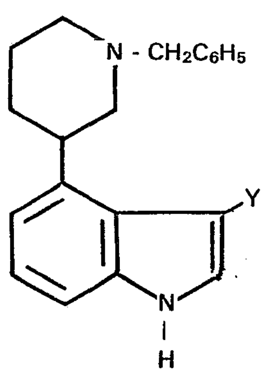

$(I_A)$

zu erhalten, worin Y die angegebene Bedeutung besitzt, das man gewünschtenfalls mit einem Halogenid der Formel III

$Hal_1$ - R'

umsetzt, worin $Hal_1$ ein Chlor-, Brom- oder Jodatom bedeutet und R' die Bedeutung von R mit Ausnahme von Wasserstoff besitzt, um ein Produkt der Formel $I_C$

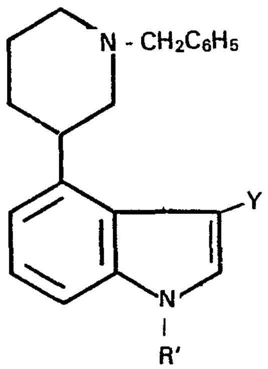

($I_C$)

zu erhalten, worin R' und Y die angegebene Bedeutung besitzen, danach die Produkte der Formeln $I_A$ und $I_C$ der Einwirkung eines Mittels zur Abspaltung der von dem Stickstoff des Piperidinylrings getragenen Benzylgruppe unterzieht, um ein Produkt der Formel $I_E$

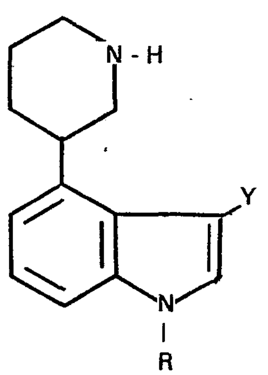

($I_E$)

zu erhalten, worin R und Y die angegebene Bedeutung besitzen, welches Produkt der Formel $I_E$ man der Einwirkung eines Oxidationsmittels unterzieht, um ein Produkt der Formel $I_I$

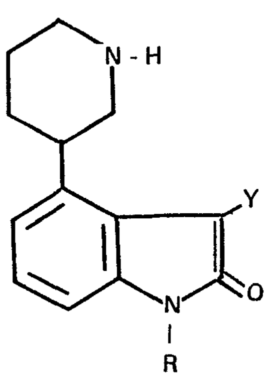

($I_I$)

zu erhalten, worin R und Y die angegebene Bedeutung besitzen, welches Produkt der Formel $I_I$ man entweder isoliert und gewünschtenfalls in ein Salz überführt oder der Einwirkung eines Mittels unterzieht, das befähigt ist, einen Rest Z' einzuführen, wobei Z' die Bedeutung von Z mit Ausnahme von derjenigen von Wasserstoff besitzt, um ein Produkt der Formel $I_J$

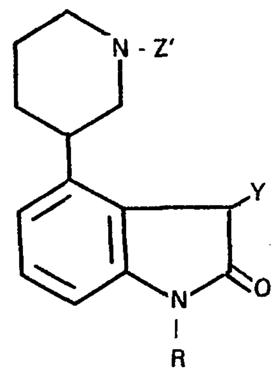

($I_J$)

zu erhalten, worin R, Y und Z' die angegebene Bedeutung besitzen, welches man isoliert und gewünschtenfalls in ein Salz überführt.

2. Verfahren gemäss Anspruch 1, dadurch gekennzeichnet, dass das Oxidationsmittel ein Dimethylsulfoxid-konzentrierte HCl-Gemisch ist.

3. Variante des Verfahrens gemäss Anspruch 1, dadurch gekennzeichnet, dass man das Produkt der Formel $I_E$ der Einwirkung eines Mittels unterzieht, das befähigt ist, einen Rest Z' einzuführen, wobei Z' die Bedeutung von Z mit Ausnahme von Wasserstoff besitzt, um ein Produkt der Formel $I_G$:

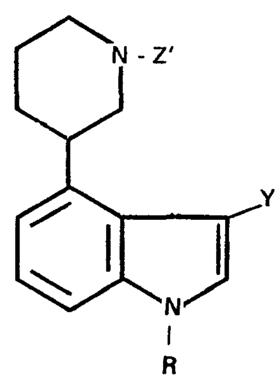

($I_G$)

zu erhalten, worin R, X', Y und Z' die angegebene Bedeutung besitzen, das man der Einwirkung eines Oxidationsmittels unterzieht, um das Produkt der Formel $I_J$ zu erhalten.

4. Verfahren gemäss einem der Ansprüche 1 bis 3, dadurch gekennzeichnet, dass man Derivate der Formel I sowie deren Additionssalze mit Mineral- oder organischen Säuren herstellt, in deren Formel I Y einen Methylrest bedeutet.

5. Verfahren gemäss einem der Ansprüche 1 bis 4, dadurch gekennzeichnet, dass man Derivate der Formel I sowie deren Additionssalze mit Mineral- oder organischen Säuren herstellt, in deren Formel I Z ein Wasserstoffatom oder einen Alkylrest mit 1 bis 8 Kohlenstoffatomen bedeutet.

6. Verfahren gemäss einem der Ansprüche 1 bis 3, dadurch gekennzeichnet, dass man das 1,3-Dihydro-3-methyl-4-(piperidin-3-yl)-2H-indol-2-on sowie dessen Additionssalze mit Mineral- oder organischen Säuren herstellt.

**Claims for the contracting states:**
BE, CH, DE, GB, IT, LI, LU, NL, SE

1. New derivatives of piperidin-3-yl indole as well as their salts of addition with mineral or organic acids, characterized in that they answer to the general formula I:

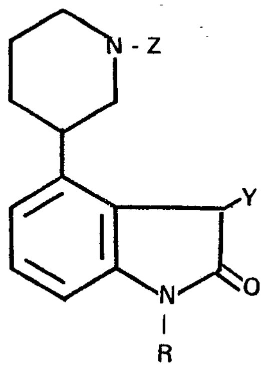

(I)

in which:
— R represents a hydrogen atom, an alkyl radical containing from 1 to 8 carbon atoms or an aralkyl radical containing from 7 to 12 carbon atoms,
— Y represents an alkyl radical containing from 1 to 8 carbon atoms,
— Z represents a hydrogen atom, a methyl radical, an alkyl or hydroxyalkyl radical containing from 2 to 8 carbon atoms, an aryloxyalkyl radical containing from 7 to 12 carbon atoms, an aralkyl radical containing from 7 to 12 carbon atoms possibly substituted by one or more halogen atoms, by one or more alkoxy radicals containing from 1 to 4 carbon atoms, by one or more alkyl radicals containing from 1 to 4 carbon atoms or by one or more OH, $CF_3$, $OCF_3$, $NO_2$, or $NH_2$ radicals or Z represents a cycloalkylalkyl radical containing from 4 to 12 carbon atoms, an alkenyl radical containing from 3 to 8 carbon atoms or an alkynyl radical containing from 3 to 8 carbon atoms.

2. New derivatives of piperidin-3-yl indole answering to the formula I as defined in claim 1 as well as their salts of addition with mineral or organic acids, characterized in that Y represents a methyl radical.

3. New derivatives of piperidin-3-yl indole according to claim 2 as well as their salts of addition with mineral or organic acids, characterized in that Z represents a hydrogen atom or an alkyl radical containing from 1 to 8 carbon atoms.

4. 1,3-dihydro-3-methyl-4-(piperidin-3-yl)2H indole-2-one, as well as its salts of addition with the mineral or organic acids.

5. Preparation process for derivatives of piperidin-3-yl indole with the formula I as defined in claim 1 as well as their salts of addition with mineral or organic acids characterized in that a product with the formula II:

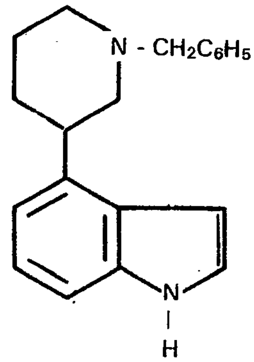

(II)

is submitted to an alkylation, so as to obtain a product with the formula $I_A$:

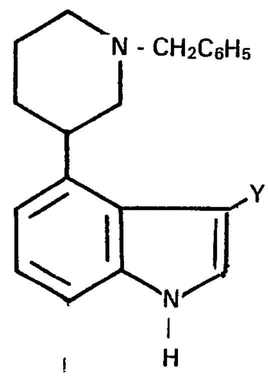

($I_A$)

in which Y has the significance already indicated, which if desired is made to react with a halogenide with the formula III:

$Hal_1 - R'$

in which $Hal_1$ represents a chlorine, bromine or iodine atom, and R' has the significance of R except for hydrogen, so as to obtain a product with the formula $I_C$:

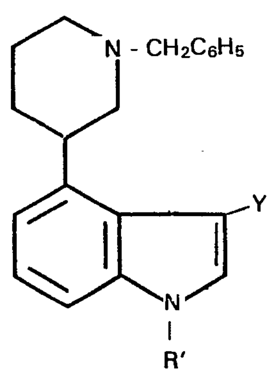

($I_C$)

in which R' and Y have the significance already indicated, then the said products with the formula $I_A$ and $I_C$ are submitted to the action of a cleavage agent for the benzyl group carried by the nitrogen of the piperidinyl ring, so as to obtain a product with the formula $I_E$:

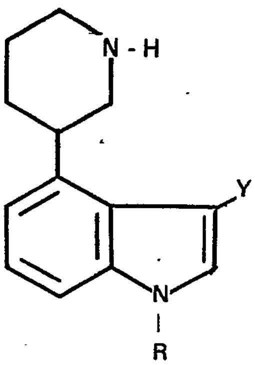

$(I_E)$

in which R and Y have the significance already indicated, which product with the formula $I_E$ is submitted to the action of an oxidizing agent, so as to obtain a product with the formula $I_I$:

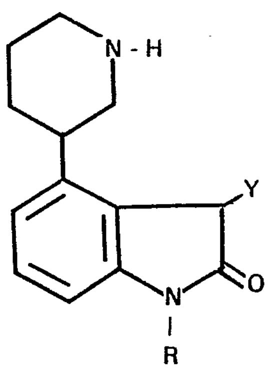

$(I_I)$

in which R and Y have the significance already indicated, which product with the formula $I_I$ is either isolated and salified if desired or submitted to the action of an agent capable of introducing a Z′ radical, Z′ having the significance of Z with the exception of hydrogen, so as to obtain a product with the formula $I_J$:

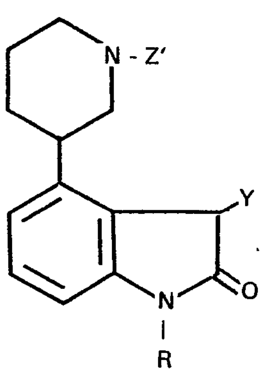

$(I_J)$

in which R, Y and Z′ have the significance already indicated, which is isolated and salified if desired.

6. Process according to claim 5, characterised in that:
— the oxidizing agent is a mixture of dimethylsulphoxide and concentrated HCl.

7. Variant of the process of claim 5, characterized in that the product with the formula $I_E$ is submitted to the action of an agent capable of introducing a radical Z′, Z′ having the significance of Z with the exception of hydrogen, so as to obtain a product with the formula $I_G$:

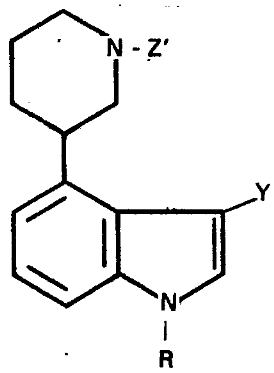

$(I_G)$

in which R, Y and Z′ have the significance already indicated, which is submitted to the action of an oxidizing agent, so as to obtain the product with the formula $I_J$.

8. Medicaments, characterized in that they are composed of the new derivatives of piperidin-3-yl indole, as defined by formula I of claim 1 as well as their salts of addition with pharmaceutically acceptable acids.

9. Medicaments, characterized in that they are composed of the new derivatives of piperidin-3-yl indole, as defined in one of the claims 2 or 3, as well as their salts of addition with pharmaceutically acceptable acids.

10. Medicaments, characterized in that they are composed of the derivative of piperidin-3-yl indole, as defined in claim 4 as well as by its salts of addition with the pharmaceutically acceptable acids.

11. Pharmaceutical compositions, characterized in that they contain, as active principle, at least one of the medicaments, as defined in any of the claims 8, 9 or 10.

**Claims for the contracting state:** AT

1. Preparation process for preparing new derivatives of piperidin-3-yl indole as well as their salts of addition with the mineral or organic acids, answering to the general formula I:

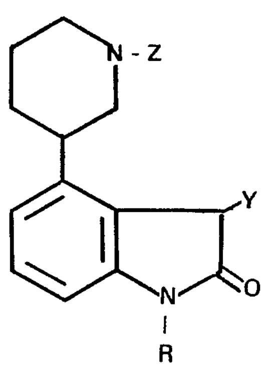

(I)

in which:
— R represents a hydrogen atom, an alkyl radical

containing from 1 to 8 carbon atoms or an aralkyl radical containing from 7 to 12 carbon atoms,

— Y represents an alkyl radical containing from 1 to 8 carbon atoms,

— Z represents a hydrogen atom, a methyl radical, an alkyl or hydroxyalkyl radical containing from 2 to 8 carbon atoms, an aryloxyalkyl radical containing from 7 to 12 carbon atoms, an aralkyl radical containing from 7 to 12 carbon atoms possibly substituted by one or more halogen atoms, by one or more alkoxy radicals containing from 1 to 4 carbon atoms, by one or more alkyl radicals containing from 1 to 4 carbon atoms or by one or more OH, $CF_3$, $OCF_3$, $NO_2$, or $NH_2$ radicals or Z represents a cycloalkylalkyl radical containing from 4 to 12 carbon atoms, an alkenyl radical containing from 3 to 8 carbon atoms or an alkynyl radical containing from 3 to 8 carbon atoms, characterized in that a product with the formula II:

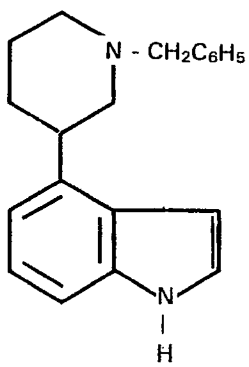

(II)

is submitted to an alkylation, so as to obtain a product with the formula $I_A$:

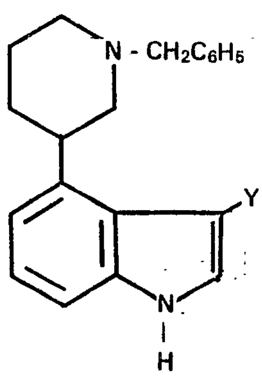

($I_A$)

in which Y has the significance already indicated, which if desired is made to react with a halogenide with the formula III:

$Hal_1$ - R'

in which $Hal_1$ represents a chlorine, bromine or iodine atom, and R' has the significance of R except for hydrogen, so as to obtain a product with the formula $I_C$:

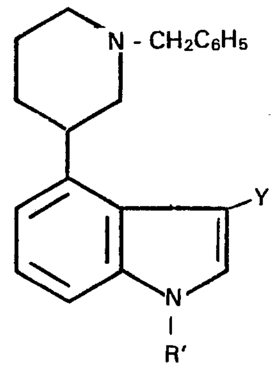

($I_C$)

in which R' and Y have the significance already indicated, then the said products with the formulae $I_A$ and $I_C$ are submitted to the action of a cleavage agent for the benzyl group carried by the nitrogen of the piperidinyl ring, so as to obtain a product with the formula $I_E$:

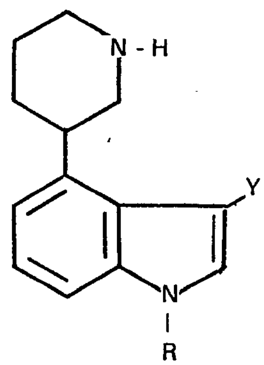

($I_E$)

in which R and Y have the significance already indicated, which product with the formula $I_E$ is submitted to the action of an oxidizing agent, so as to obtain a product with the formula $I_I$:

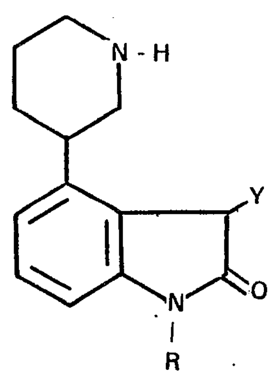

($I_I$)

in which R and Y have the significance already indicated, which product with the formula $I_I$ is either isolated and salified if desired or submitted to the action of an agent capable of introducing a radical Z', Z' having the significance of Z with the exception of hydrogen, so as to obtain a product with the formula $I_J$:

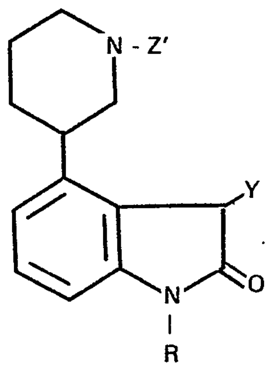

($I_J$)

in which R, Y and Z' have the significance already indicated, which is isolated and salified if desired.

2. Process according to claim 1, characterized in that:

— the oxidizing agent is a mixture of dimethylsulphoxide and concentrated HCl.

3. Variant of the process of claim 1, characterized in that the product with the formula $I_E$ is submitted to the action of an agent capable of introducing a Z' radical, Z' having the significance of Z with the exception of hydrogen, so as to obtain a product with the formula $I_G$:

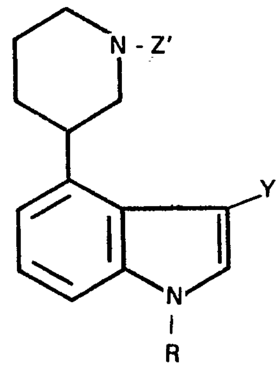

($I_G$)

in which R, X', Y and Z' have the significance already indicated, which is submitted to the action of an oxidizing agent, so as to obtain the product with the formula $I_J$.

4. Process according to any one of the claims 1 to 3, characterized in that derivatives with the formula I are prepared, as well as their salts of addition with mineral or organic acids, in which formula I, Y represents a methyl radical.

5. Process according to any one of the claims 1 to 4, characterized in that derivatives with the formula I are prepared, as well as their salts of addition with mineral or organic acids, in which formula I, Z represents a hydrogen atom or an alkyl radical containing from 1 to 8 carbon atoms.

6. Process according to any one of the claims 1 to 3, characterized in that 1,3-dihydro-3-methyl-4-(piperidin-3-yl)-2H indole-2-one, is prepared, as well as its salts of addition with mineral or organic acids.